# EUROPEAN PATENT APPLICATION

(11) **EP 2 405 006 A2**
(43) Date of publication of application: **11.01.2012**
(21) Application number: 10748904.9
(22) Date of filing: 16.02.2010
(51) Int. Cl.: C12N 15/52, C12N 1/21, C12P 13/04, C12R 1/19

(54) **MICROORGANISM WHICH PRODUCES L-AMINO ACID AND METHOD FOR PRODUCING L-AMINO ACID USING THE SAME**

(30) Priority: 03.03.2009 KR 20090018128
(71) Applicant: CJ CheilJedang Corporation, Seoul, 100-400 (KR)
(72) Inventor: JU, Jae Yeong, Seongnam-si Gyeonggi-do 463-703 (KR); LEE, Kwang Ho, Daejeon 305-755 (KR); BAE, Hyun Ae, Seoul 157-894 (KR)
(74) Representative: Vossius & Partner
(86) International application number: PCT/KR2010/000933
(87) International publication number: WO 2010/101360

(57) **Abstract**

The present invention relates to a microorganism belonging to the genus *Escherichia* sp. and a method for producing L-amino acid using the same. The microorganism belonging to the genus *Escherichia* sp. with sucrose assimilability and L-amino acid producing ability is obtained by introducing a gene encoding a sucrose assimilative microorganism-derived sucrose metabolic enzyme to the sucrose non-assimilative microorganism belonging to the genus *Escherichia* sp. having an L-amino acid producing ability.

<110>    CJ CheilJedang Corporation

<120>    L-amino acid producing microorganisms and process for producing
         L-amino acids using the same

<160>    8

<170>    KopatentIn 1.71

<210>    1
<211>    30
<212>    DNA
<213>    Artificial Sequence

<220>
<223>    Primer for amplification of scrKABR from Streptococcus mutans


<400>    1
cgccgtgcac tgccgaacag ttctctatgc
30


<210>    2
<211>    36
<212>    DNA
<213>    Artificial Sequence

<220>
<223>    Primer for amplification of scrKYABR from Streptococcus mutans


<400>    2
caccaggcct actagtctgg ctggacgctt tctcga
36


<210>    3
<211>    6472
<212>    DNA
<213>    Streptococcus mutans (ATCC700610)

<220>
<221>    gene
<222>    (1)..(6472)
<223>    polynucleotide including scrKABR from Streptococcus mutans


<400>    3
gtgcactgcc gaacagttct ctatgctctg catataattc atctaagggg acacctttat
60

aaacaccact tttaacaaca gacactccat taggatgcgc agaaatcgcc caatattctc
120

ccgtcgtttc actaggaatg tcataaccaa attcttttct gagccgattg ccgccccaga
180

tttttttatg catttgtgat tgtaaaaaca aaggttctgc catagtgttc tcctttctgt
240

ttttcaatat accactatct agtttcagag gaaatcaaga gtaattatat tcacttcatt
300

atcctcataa aatgaaaatt tcgttcacgt aaattctaac agtttaatat tgaatattag
360

aaggtgcttt aatcttccaa ttcattttta attaacgcgc tgctatcttt ttagctaaag
420

cgagatttcc caatgttgct gaaccatttt ctgcaacagc tggtgtcaca atataatctt
480

taacatctgg aactggaaga tagtcattca aaagtgaagt aaatttttca cgaacccgat
540

tgagcatatg ttcttgtgcc ataacgcctc cgccaaatac aatgacttgc ggacgataaa
600

ggacagtcgc ttgaatagcc gcctgagcaa tgtagtatgc ctgaatatcc caaacttctg
660

agttttgctc aattaactca ccacgaatac cagtacgagc ctctaagcta ggacccgctg
720

caagtccttc taaacagcct ttatggaaag gacaggtgcc tacaaaacca tgatgaacat
780

cattgggatg cggagccatg taaacgtgtc cagcttccgt atgtcccata ccgccaatga
840

attcgccatt ttgaatagtc cctgctccaa tgcctgttcc aatagtataa taaaccagac
900

ttttaacatt tgaacgagca attgtttccc cataagcaga agaattaaca tctgtcgtaa
960

agtaaaatgg aattttaaaa tctttagaaa ttaagccgac aaaatcaacg ttagcccagt
1020

ttggctttgg tgttgaagta atgtaaccat aagtgtctga attttgatca atatcaatag
1080

ggccaaaaga accaatggca acactggcta aatcagcttc aaatttttta aagaaagcaa
1140

ctgttttttc tattgtttca taaggtgttg ttgttgggaa ctgaactttt tctaaaattt
1200

gaaaattttc atcacctaca gcacagacaa attttgttcc gccagcttcg atgctgccat
1260

ataatttaga cataataaac ctcttatatt ttacttaact taccaaatca atttttggat
1320

ttgaataccc ttaatctacc cttaaggact acttaacatt atatcataat ttgctaaatt
1380

tcaagtatta gttagccaaa aggctttaat actattgtta ctgaatcttt taagcagtca
1440

aaaagacctt acggtctttc tgataatatt tcttattttt taacttcaag taagctatca
1500

cctacagcaa cagttcctga agaagcgagg gtttcaactg acgcataatc ggctgtatta
1560

gtaacaatga acattgttgt attatcaaga ccagcttctg caattttgtc actatcaaat
1620

gttccgagaa cgtcaccttt tttaattttt tgatctgctt gaactttttg ttcaaatcct
1680

ttaccttcca ttgatactgt atcaatacca atatgaataa gaatctccgc accattgtct
1740

gacttaatac cataagcatg gccagtatca aaagcaattt gaacagtccc atcaaccggt
1800

gcataaactg tattaccact aggtttgata gcaatcccct tacccattgc ttcgctagaa
1860

aaaacagggt cattaacaga ggttaattca acagcttcac cagcaagagg agccgcaaga
1920

acttcatcag taacttgtgc cttatttgct gcagaagcag cttcttctgg aatttcttgc
1980

acttcttcaa tagcttcttc gacagcagct tcagcagcaa agacatcaac agccttcgtc
2040

ttaccatagc cataagttac tgcaaaagct atagcaaaac tgattaattc acagactaca
2100

taaaatggaa tagaacttgc cttaatagaa aggaaaccaa gaaaacctgc cgaaccaagg
2160

gaaaccgcta caacttgaag taagcccgca attgctgcag cactagctga gccaatcaga
2220

gcacaaaaga atgggaaacg atatttcaaa ttcaccccaa aaagagcagg ttctgtaata
2280

ccaagaagcg ctgacacacc tgaagaagat gagagacctt tcatcttctt atcttttgtt
2340

aagaagtaaa ttgcaaaggt tgctgcgcct tgtgcgacat tggccattga agctgtaaca
2400

aagataaagt caccatgacc agtaccattt tggaaagctg aaatgagttg ggtttcaata
2460

gctgggaaac tttgatggag accagtcata actacaggtg aatacagagc accaaagaca
2520

cccataccta ggaatcccgt tgtatcatac agccatacga ttccatttgt aagccagtca
2580

gaaacttctt tcataacagg accaataacg ataaatgtca aaaatccagt aataataact
2640

gacaataaag gtgtaaaggt aaagtcaact gctgagggca atctcttatg gaaaaatttt
2700

tctaagatag ataaaagcca tacagccaca agtactggaa tcacttgata agtataactt
2760

gcctgagtaa catgcaaccc aaaaatattc caaaatcctg tataagcacc aatagtagca
2820

gcctttgaaa ttggagcatt agctgctaaa ccaatgatat ttgctgcccc cggtgcgacc
2880

ataatcatgc cgattgaagc tcctaagaat tggttagcac caaaacgttt agctgctgaa
2940

atcccaacca aaatgggtaa gaaccagaaa ggcgctgctg acattaattg aatcatgtca
3000

gatgaaccct taataatcgg gaattgttga accagtgact tagtaccaaa taaaccttct
3060

gaagttaaaa agttatttaa agccattaag agaccgcctg ctaccaaggc agggataatt
3120

ggtacaaaga tatcagacaa cagtttgatc aaagccataa taggattgaa ctttttacca
3180

ctggcagcaa tctttttcaa atcatctgtt gagacttctg ttaaacctgt ttgtttaata
3240

atttcatcat aaacaaagtt aacatcacca gggccaataa taacttgata ttgaccatcc
3300

gttttaaaag ttcccttgac atcagcattt ttatccaacg ctttttgatc tactttactg
3360

tcatctttta aaacaagacg aagacgtgtc gcacagtgag cggcagctac aagattatct
3420

ttaccaacag ctgtgatgac ttcactagct actttgctat aatccatttg caaaaaactc
3480

cttaataatt attttgtttt gaaagcgttt ttttactttc cacgttattc attttatcat
3540

ttttttccaa tatgtcaatc gttttacata aaattctttt ttgcaacaaa agattgattt
3600

ttagaaagaa aacgtttact atattagtat gtagataaac tattattagg agtagataaa
3660

cgatgaattt gcctcaaaat atcagatatc gccgctatca agattggact gaagaagaaa
3720

taaaaagtat taaaaccaat gtagctttgt ctccttggca tacaacgtat catatagaac
3780

ctaaaacagg actccttaat gatccaaacg gttttcccta ttttaatgga aaatttaacc
3840

ttttttatca aaattggcca tttggagcag ctcacggctt aaaatcttgg atccatactg
3900

aaagtgaaga cttagtccat ttcaaagaaa caggtacagt cctttatccc gatacttccc
3960

atgacagcca tggtgcatac tcgggcagtg cctatgaaat cggtgatcag ctttttctct
4020

tttatacagg aaatgtccga gatgaaaatt gggttcgtca tccacttcaa atcggcgctt
4080

ttatggataa aaaaggtaat atccaaaaat ttactgatgt ccttattaaa cagccaaatg
4140

atgttactga acactttcgc gatccccaaa tttttaatta taaaggacaa ttttacgcta
4200

ttgttggagc acaaagtcta gataaaaaag gatttatcaa actctataaa gctgttgata
4260

atgatattaa gaattggcaa gaagttggta atctagactt tggcggaagt aaatctgagt
4320

atatgattga gtgcccaaat cttgttttta taaacgaaca gcctgtcctg atttatagtc
4380

ctcagggact cagtaaatct gaattagatt atcataatat ttatcctaat acttacaaag
4440

tatgtcaatc gtttgacaca gaaaagcctg ccctagttga tgcatcggaa attcaaaatc
4500

ttgacttcgg atttgaatgt tatgctaccc aagctttcaa tgctcctgat ggccgtgttt
4560

atgctgtctc atggattggg ttaccagata ttgattatcc aagtgattca tacgactatc
4620

aaggagcttt gagcctcgtc aaagagctaa gcctaaaaca cggtaaactc tatcaatatc
4680

ccgttgaagc tgttcgttca ttacgttctg aaaaagaagc agtcacttac aagccagaaa
4740

ccaataatac ttatgaatta gagttaactt ttgactcttc atcagttaat gaattgctcc
4800

tttttgctga taataaaggc aatggtctag ctattacagt tgatactaag atgggaacca
4860

ttctgatcga tcgctctaaa gcaggggagc aatatgcctt agaatttgga agccaacgtt
4920

cttgctctat ccaagcaaaa gagactgttg tcaatatttt tgttgacaaa tcgatttttg
4980

aaatttttat taataaggga gaaaaagttt ttactggacg tgtttttcca aatgacaaac
5040

aaactggtat tgtgattaaa tctggaaagc caagcggtaa ttactacgaa ttgaaatatt
5100

aactatggtt gcaaaattaa cagatgtcgc aaaactcgct ggcgtaagtc caacaaccgt
5160

ttcacgcgtg attaatcgaa aaggctatct ctctgaaaaa acaattacta aagtacaggc
5220

tgccatgaaa actctaggat acaagcccaa taatctcgct cgcagccttc aggggaaatc
5280

tgccaagcta attggactta ttttccctaa tatcagtcac atcttctatt ctgaacttat
5340

tgaatattta gaaatagagt tgtttaaaca tggctacaaa gccattattt gtaacagtca
5400

gaataatccc gataaagaac gggattatct cgaaatgtta gaagctaatc aggttgatgg
5460

cattatttcc agtagtcaca acctcggcat tgatgactat gagaaagttt ctgctcctat
5520

tattgccttt gatcgtaact tagcacccaa tattcccatc gtctcttctg acaattttga
5580

aggcggacga atggccgcaa aacttttaaa aaaacacggc tgccaacatc ccattatgat
5640

cgctggaaaa gataattcta attctcggac aggactgcga caattaggct ttaagtccgt
5700

ctttgctcaa gcacctattt ttcatctatc tggagagctg tccatcattc gtaaagaaat
5760

ggaaataaaa gtaattctcc aaaatgaaaa acctgatggt atctttttgt ccgatgatat
5820

gacagctatt ttaacaatga aaattgctaa ccagttaaac attaccattc cccatgaact
5880

taaaattatt gggtacgatg gaacacactt tgttgagaac tactatcctt atctaacaac
5940

tattaggcaa cctattaaag atatcgcaca tcttttggta gacgtattgc taaagaaaat
6000

tgatcaccaa gatactccta aagattatat tctccccgtt ggtcttttat caggagaaag
6060

tgtttagaat caaaaggttt atttaggttt gtcttttgtg ggttgattaa aagtccttac
6120

ctaattaatg agcatcagtt caatgtcaat atttaatcaa ctttgcgcaa aagaatagac
6180

atacaattct tagttttaaa ttcaaaaaga agaggtaaga tttaagccta aaatcttacc
6240

tcttctttta tcagccctta ttattttctt tcaagcacaa actgacttaa aacgccatta
6300

ataaacttac tggatttctc atctgaatac ttttttgcaa tttcaataat ttcattaaga
6360

gcaacacgtt ctggtgtttc cttatgatat ttaatttcgt ataaacctaa acgcaacagg
6420

ctcttatcaa tcaaagtcaa tcgagaaagc gtccagccag actagtaggg ca
6472

<210>    4
<211>    882
<212>    DNA
<213>    Streptococcus mutans (ATCC700610)

<220>
<221>    gene
<222>    (1)..(882)
<223>    scrK

<400>    4
atgtctaaat tatatggcag catcgaagct ggcggaacaa aatttgtctg tgctgtaggt
60

gatgaaaatt ttcaaatttt agaaaaagtt cagttcccaa caacaacacc ttatgaaaca
120

atagaaaaaa cagttgcttt ctttaaaaaa tttgaagctg atttagccag tgttgccatt
180

ggttcttttg gccctattga tattgatcaa aattcagaca cttatggtta cattacttca
240

acaccaaagc caaactgggc taacgttgat tttgtcggct taatttctaa agattttaaa
300

attccatttt actttacgac agatgttaat tcttctgctt atggggaaac aattgctcgt
360

tcaaatgtta aaagtctggt ttattatact attggaacag gcattggagc agggactatt
420

caaaatggcg aattcattgg cggtatggga catacggaag ctggacacgt ttacatggct
480

ccgcatccca atgatgttca tcatggtttt gtaggcacct gtcctttcca taaaggctgt
540

ttagaaggac ttgcagcggg tcctagctta gaggctcgta ctggtattcg tggtgagtta
600

attgagcaaa actcagaagt ttgggatatt caggcatact acattgctca ggcggctatt
660

caagcgactg tcctttatcg tccgcaagtc attgtatttg gcggaggcgt tatggcacaa

720

gaacatatgc tcaatcgggt tcgtgaaaaa tttacttcac ttttgaatga ctatcttcca
780

gttccagatg ttaaagatta tattgtgaca ccagctgttg cagaaaatgg ttcagcaaca
840

ttgggaaatc tcgctttagc taaaaagata gcagcgcgtt aa
882

<210> 5
<211> 1995
<212> DNA
<213> Streptococcus mutans (ATCC700610)

<220>
<221> gene
<222> (1)..(1995)
<223> scrA

<400> 5
atggattata gcaaagtagc tagtgaagtc atcacagctg ttggtaaaga taatcttgta
60

gctgccgctc actgtgcgac acgtcttcgt cttgttttaa aagatgacag taaagtagat
120

caaaaagcgt tggataaaaa tgctgatgtc aagggaactt ttaaaacgga tggtcaatat
180

caagttatta ttggccctgg tgatgttaac tttgtttatg atgaaattat taaacaaaca
240

ggtttaacag aagtctcaac agatgatttg aaaaagattg ctgccagtgg taaaaagttc
300

aatcctatta tggctttgat caaactgttg tctgatatct ttgtaccaat tatccctgcc
360

ttggtagcag gcggtctctt aatggcttta aataactttt taacttcaga aggtttattt
420

ggtactaagt cactggttca acaattcccg attattaagg gttcatctga catgattcaa
480

ttaatgtcag cagcgccttt ctggttctta cccattttgg ttgggatttc agcagctaaa
540

cgttttggtg ctaaccaatt cttaggagct tcaatcggca tgattatggt cgcaccgggg
600

gcagcaaata tcattggttt agcagctaat gctccaattt caaaggctgc tactattggt
660

gcttatacag gatttggaaa tatttttggg ttgcatgtta ctcaggcaag ttatacttat
720

caagtgattc cagtacttgt ggctgtatgg cttttatcta tcttagaaaa atttttccat
780

aagagattgc cctcagcagt tgactttacc tttacacctt tattgtcagt tattattact
840

ggatttttga catttatcgt tattggtcct gttatgaaag aagtttctga ctggcttaca
900

aatggaatcg tatggctgta tgatacaacg ggattcctag gtatgggtgt ctttggtgct
960

ctgtattcac ctgtagttat gactggtctc catcaaagtt tcccagctat tgaaacccaa
1020

ctcatttcag ctttccaaaa tggtactggt catggtgact ttatctttgt tacagcttca
1080

atggccaatg tcgcacaagg cgcagcaacc tttgcaattt acttcttaac aaaagataag
1140

aagatgaaag gtctctcatc ttcttcaggt gtgtcagcgc ttcttggtat tacagaacct
1200

gctctttttg gggtgaattt gaaatatcgt ttcccattct tttgtgctct gattggctca
1260

gctagtgctg cagcaattgc gggcttactt caagttgtag cggtttccct tggttcggca
1320

ggttttcttg gtttcctttc tattaaggca agttctattc cattttatgt agtctgtgaa
1380

ttaatcagtt ttgctatagc ttttgcagta acttatggct atggtaagac gaaggctgtt
1440

gatgtctttg ctgctgaagc tgctgtcgaa gaagctattg aagaagtgca agaaattcca
1500

gaagaagctg cttctgcagc aaataaggca caagttactg atgaagttct tgcggctcct
1560

cttgctggtg aagctgttga attaacctct gttaatgacc ctgttttttc tagcgaagca
1620

atgggtaagg ggattgctat caaacctagt ggtaatacag tttatgcacc ggttgatggg
1680

actgttcaaa ttgcttttga tactggccat gcttatggta ttaagtcaga caatggtgcg
1740

gagattctta ttcatattgg tattgataca gtatcaatgg aaggtaaagg atttgaacaa
1800

aaagttcaag cagatcaaaa aattaaaaaa ggtgacgttc tcggaacatt tgatagtgac
1860

aaaattgcag aagctggtct tgataataca acaatgttca ttgttactaa tacagccgat
1920

tatgcgtcag ttgaaaccct cgcttcttca ggaactgttg ctgtaggtga tagcttactt
1980

gaagttaaaa aataa
1995

<210> 6
<211> 1440
<212> DNA
<213> Streptococcus mutans (ATCC700610)

<220>
<221> gene
<222> (1)..(1440)
<223> scrB

<400> 6
atgaatttgc ctcaaaatat cagatatcgc cgctatcaag attggactga agaagaaata
60

aaaagtatta aaaccaatgt agctttgtct ccttggcata caacgtatca tatagaacct
120

aaaacaggac tccttaatga tccaaacggt ttttcctatt ttaatggaaa atttaacctt
180

ttttatcaaa attggccatt tggagcagct cacggcttaa aatcttggat ccatactgaa
240

agtgaagact tagtccattt caaagaaaca ggtacagtcc tttatcccga tacttcccat
300

gacagccatg gtgcatactc gggcagtgcc tatgaaatcg gtgatcagct tttctcttt
360

tatacaggaa atgtccgaga tgaaaattgg gttcgtcatc cacttcaaat cggcgctttt
420

atggataaaa aaggtaatat ccaaaaattt actgatgtcc ttattaaaca gccaaatgat
480

gttactgaac actttcgcga tccccaaatt tttaattata aaggacaatt ttacgctatt
540

gttggagcac aaagtctaga taaaaaagga tttatcaaac tctataaagc tgttgataat
600

gatattaaga attggcaaga agttggtaat ctagactttg gcggaagtaa atctgagtat
660

atgattgagt gcccaaatct tgtttttata aacgaacagc ctgtcctgat ttatagtcct
720

cagggactca gtaaatctga attagattat cataatattt atcctaatac ttacaaagta
780

tgtcaatcgt ttgacacaga aaagcctgcc ctagttgatg catcggaaat tcaaaatctt
840

gacttcggat ttgaatgtta tgctacccaa gctttcaatg ctcctgatgg ccgtgtttat
900

gctgtctcat ggattgggtt accagatatt gattatccaa gtgattcata cgactatcaa
960

ggagctttga gcctcgtcaa agagctaagc ctaaaacacg gtaaactcta tcaatatccc
1020

gttgaagctg ttcgttcatt acgttctgaa aaagaagcag tcacttacaa gccagaaacc
1080

aataatactt atgaattaga gttaactttt gactcttcat cagttaatga attgctcctt
1140

tttgctgata ataaaggcaa tggtctagct attacagttg atactaagat gggaaccatt
1200

ctgatcgatc gctctaaagc aggggagcaa tatgccttag aatttggaag ccaacgttct
1260

tgctctatcc aagcaaaaga gactgttgtc aatatttttg ttgacaaatc gatttttgaa
1320

atttttatta ataagggaga aaaagttttt actggacgtg tttttccaaa tgacaaacaa
1380

actggtattg tgattaaatc tggaaagcca agcggtaatt actacgaatt gaaatattaa
1440


1440


<210>    7
<211>    963
<212>    DNA
<213>    Streptococcus mutans (ATCC700610)

<220>
<221>    gene
<222>    (1)..(963)
<223>    scrR


<400>    7
atggttgcaa aattaacaga tgtcgcaaaa ctcgctggcg taagtccaac aaccgtttca
60

cgcgtgatta atcgaaaagg ctatctctct gaaaaaacaa ttactaaagt acaggctgcc
120

atgaaaactc taggatacaa gcccaataat ctcgctcgca gccttcaggg gaaatctgcc
180

aagctaattg gacttatttt ccctaatatc agtcacatct tctattctga acttattgaa
240

tatttagaaa tagagttgtt taaacatggc tacaaagcca ttatttgtaa cagtcagaat

300

aatcccgata aagaacggga ttatctcgaa atgttagaag ctaatcaggt tgatggcatt
360

atttccagta gtcacaacct cggcattgat gactatgaga aagtttctgc tcctattatt
420

gcctttgatc gtaacttagc acccaatatt cccatcgtct cttctgacaa ttttgaaggc
480

ggacgaatgg ccgcaaaact tttaaaaaaa cacggctgcc aacatcccat tatgatcgct
540

ggaaaagata attctaattc tcggacagga ctgcgacaat taggctttaa gtccgtcttt
600

gctcaagcac ctatttttca tctatctgga gagctgtcca tcattcgtaa agaaatggaa
660

ataaaagtaa ttctccaaaa tgaaaaacct gatggtatct ttttgtccga tgatatgaca
720

gctattttaa caatgaaaat tgctaaccag ttaaacatta ccattcccca tgaacttaaa
780

attattgggt acgatggaac acactttgtt gagaactact atccttatct aacaactatt
840

aggcaaccta ttaaagatat cgcacatctt ttggtagacg tattgctaaa gaaaattgat
900

caccaagata ctcctaaaga ttatattctc cccgttggtc ttttatcagg agaaagtgtt
960

tag
963

<210> 8
<211> 9960
<212> DNA
<213> Artificial Sequence

<220>
<223> pAscrSM

<400> 8
tgccgaacag ttctctatgc tctgcatata attcatctaa ggggacacct ttataaacac
60

cacttttaac aacagacact ccattaggat gcgcagaaat cgcccaatat tctcccgtcg
120

tttcactagg aatgtcataa ccaaattctt ttctgagccg attgccgccc cagatttttt
180

tatgcatttg tgattgtaaa aacaaaggtt ctgccatagt gttctccttt ctgtttttca
240

atataccact atctagtttc agaggaaatc aagagtaatt atattcactt cattatcctc
300

ataaaatgaa aatttcgttc acgtaaattc taacagttta atattgaata ttagaaggtg
360

ctttaatctt ccaattcatt tttaattaac gcgctgctat ctttttagct aaagcgagat
420

ttcccaatgt tgctgaacca ttttctgcaa cagctggtgt cacaatataa tctttaacat
480

ctggaactgg aagatagtca ttcaaaagtg aagtaaattt ttcacgaacc cgattgagca
540

tatgttcttg tgccataacg cctccgccaa atacaatgac ttgcggacga taaaggacag
600

tcgcttgaat agccgcctga gcaatgtagt atgcctgaat atcccaaact tctgagtttt
660

gctcaattaa ctcaccacga ataccagtac gagcctctaa gctaggaccc gctgcaagtc
720

cttctaaaca gcctttatgg aaaggacagg tgcctacaaa accatgatga acatcattgg
780

gatgcggagc catgtaaacg tgtccagctt ccgtatgtcc cataccgcca atgaattcgc
840

cattttgaat agtccctgct ccaatgcctg ttccaatagt ataataaacc agacttttaa
900

catttgaacg agcaattgtt tccccataag cagaagaatt aacatctgtc gtaaagtaaa
960

atggaatttt aaaatcttta gaaattaagc cgacaaaatc aacgttagcc cagtttggct
1020

ttggtgttga agtaatgtaa ccataagtgt ctgaattttg atcaatatca atagggccaa
1080

aagaaccaat ggcaacactg gctaaatcag cttcaaattt tttaaagaaa gcaactgttt
1140

tttctattgt ttcataaggt gttgttgttg ggaactgaac tttttctaaa atttgaaaat
1200

tttcatcacc tacagcacag acaaattttg ttccgccagc ttcgatgctg ccatataatt
1260

tagacataat aaacctctta tattttactt aacttaccaa atcaattttt ggatttgaat
1320

acccttaatc tacccttaag gactacttaa cattatatca taatttgcta aatttcaagt
1380

attagttagc caaaaggctt taatactatt gttactgaat cttttaagca gtcaaaaaga
1440

ccttacggtc tttctgataa tatttcttat tttttaactt caagtaagct atcacctaca
1500

gcaacagttc ctgaagaagc gagggtttca actgacgcat aatcggctgt attagtaaca
1560

atgaacattg ttgtattatc aagaccagct tctgcaattt tgtcactatc aaatgttccg
1620

agaacgtcac cttttttaat tttttgatct gcttgaactt tttgttcaaa tcctttacct
1680

tccattgata ctgtatcaat accaatatga ataagaatct ccgcaccatt gtctgactta
1740

ataccataag catggccagt atcaaaagca atttgaacag tcccatcaac cggtgcataa
1800

actgtattac cactaggttt gatagcaatc cccttaccca ttgcttcgct agaaaaaaca
1860

gggtcattaa cagaggttaa ttcaacagct tcaccagcaa gaggagccgc aagaacttca
1920

tcagtaactt gtgccttatt tgctgcagaa gcagcttctt ctggaatttc ttgcacttct
1980

tcaatagctt cttcgacagc agcttcagca gcaaagacat caacagcctt cgtcttacca
2040

tagccataag ttactgcaaa agctatagca aaactgatta attcacagac tacataaaat
2100

ggaatagaac ttgccttaat agaaaggaaa ccaagaaaac ctgccgaacc aagggaaacc
2160

gctacaactt gaagtaagcc cgcaattgct gcagcactag ctgagccaat cagagcacaa
2220

aagaatggga aacgatattt caaattcacc ccaaaaagag caggttctgt aataccaaga
2280

agcgctgaca cacctgaaga agatgagaga cctttcatct tcttatcttt tgttaagaag
2340

taaattgcaa aggttgctgc gccttgtgcg acattggcca ttgaagctgt aacaaagata
2400

aagtcaccat gaccagtacc attttggaaa gctgaaatga gttgggtttc aatagctggg
2460

aaactttgat ggagaccagt cataactaca ggtgaataca gagcaccaaa gacacccata
2520

cctaggaatc ccgttgtatc atacagccat acgattccat ttgtaagcca gtcagaaact
2580

tctttcataa caggaccaat aacgataaat gtcaaaaatc cagtaataat aactgacaat
2640

aaaggtgtaa aggtaaagtc aactgctgag ggcaatctct tatggaaaaa tttttctaag
2700

atagataaaa gccatacagc cacaagtact ggaatcactt gataagtata acttgcctga
2760

gtaacatgca acccaaaaat attccaaaat cctgtataag caccaatagt agcagccttt
2820

gaaattggag cattagctgc taaaccaatg atatttgctg cccccggtgc gaccataatc
2880

atgccgattg aagctcctaa gaattggtta gcaccaaaac gtttagctgc tgaaatccca
2940

accaaaatgg gtaagaacca gaaaggcgct gctgacatta attgaatcat gtcagatgaa
3000

cccttaataa tcgggaattg ttgaaccagt gacttagtac caaataaacc ttctgaagtt
3060

aaaaagttat ttaaagccat taagagaccg cctgctacca aggcagggat aattggtaca
3120

aagatatcag acaacagttt gatcaaagcc ataataggat tgaacttttt accactggca
3180

gcaatctttt tcaaatcatc tgttgagact tctgttaaac ctgtttgttt aataatttca
3240

tcataaacaa agttaacatc accagggcca ataataactt gatattgacc atccgtttta
3300

aaagttccct tgacatcagc atttttatcc aacgcttttt gatctacttt actgtcatct
3360

tttaaaacaa gacgaagacg tgtcgcacag tgagcggcag ctacaagatt atctttacca
3420

acagctgtga tgacttcact agctactttg ctataatcca tttgcaaaaa actccttaat
3480

aattattttg ttttgaaagc gtttttttac tttccacgtt attcatttta tcattttttt
3540

ccaatatgtc aatcgtttta cataaaattc ttttttgcaa caaaagattg atttttagaa
3600

agaaaacgtt tactatatta gtatgtagat aaactattat taggagtaga taaacgatga
3660

atttgcctca aaatatcaga tatcgccgct atcaagattg gactgaagaa gaaataaaaa
3720

gtattaaaac caatgtagct ttgtctcctt ggcatacaac gtatcatata gaacctaaaa
3780

caggactcct taatgatcca aacggttttt cctattttaa tggaaaattt aacctttttt
3840

atcaaaattg gccatttgga gcagctcacg gcttaaaatc ttggatccat actgaaagtg
3900

aagacttagt ccatttcaaa gaaacaggta cagtccttta tcccgatact tcccatgaca
3960

gccatggtgc atactcgggc agtgcctatg aaatcggtga tcagcttttt ctcttttata
4020

caggaaatgt ccgagatgaa aattgggttc gtcatccact tcaaatcggc gcttttatgg
4080

ataaaaaagg taatatccaa aaatttactg atgtccttat taaacagcca aatgatgtta
4140

ctgaacactt tcgcgatccc caaattttta attataaagg acaattttac gctattgttg
4200

gagcacaaag tctagataaa aaaggattta tcaaactcta taaagctgtt gataatgata
4260

ttaagaattg gcaagaagtt ggtaatctag actttggcgg aagtaaatct gagtatatga
4320

ttgagtgccc aaatcttgtt tttataaacg aacagcctgt cctgatttat agtcctcagg
4380

gactcagtaa atctgaatta gattatcata atatttatcc taatacttac aaagtatgtc
4440

aatcgtttga cacagaaaag cctgccctag ttgatgcatc ggaaattcaa aatcttgact
4500

tcggatttga atgttatgct acccaagctt tcaatgctcc tgatggccgt gtttatgctg
4560

tctcatggat tgggttacca gatattgatt atccaagtga ttcatacgac tatcaaggag
4620

ctttgagcct cgtcaaagag ctaagcctaa aacacggtaa actctatcaa tatcccgttg
4680

aagctgttcg ttcattacgt tctgaaaaag aagcagtcac ttacaagcca gaaaccaata
4740

atacttatga attagagtta acttttgact cttcatcagt taatgaattg ctcctttttg
4800

ctgataataa aggcaatggt ctagctatta cagttgatac taagatggga accattctga
4860

tcgatcgctc taaagcaggg gagcaatatg ccttagaatt tggaagccaa cgttcttgct
4920

ctatccaagc aaaagagact gttgtcaata tttttgttga caaatcgatt tttgaaattt
4980

ttattaataa gggagaaaaa gtttttactg gacgtgtttt tccaaatgac aaacaaactg
5040

gtattgtgat taaatctgga aagccaagcg gtaattacta cgaattgaaa tattaactat
5100

ggttgcaaaa ttaacagatg tcgcaaaact cgctggcgta agtccaacaa ccgtttcacg
5160

cgtgattaat cgaaaaggct atctctctga aaaaacaatt actaaagtac aggctgccat
5220

gaaaactcta ggatacaagc ccaataatct cgctcgcagc cttcagggga aatctgccaa
5280

gctaattgga cttattttcc ctaatatcag tcacatcttc tattctgaac ttattgaata
5340

tttagaaata gagttgttta aacatggcta caaagccatt atttgtaaca gtcagaataa
5400

tcccgataaa gaacgggatt atctcgaaat gttagaagct aatcaggttg atggcattat
5460

ttccagtagt cacaacctcg gcattgatga ctatgagaaa gtttctgctc ctattattgc
5520

ctttgatcgt aacttagcac ccaatattcc catcgtctct tctgacaatt ttgaaggcgg
5580

acgaatggcc gcaaaacttt taaaaaaaca cggctgccaa catcccatta tgatcgctgg
5640

aaaagataat tctaattctc ggacaggact gcgacaatta ggctttaagt ccgtctttgc
5700

tcaagcacct atttttcatc tatctggaga gctgtccatc attcgtaaag aaatggaaat
5760

aaaagtaatt ctccaaaatg aaaaacctga tggtatcttt ttgtccgatg atatgacagc
5820

tattttaaca atgaaaattg ctaaccagtt aaacattacc attccccatg aacttaaaat
5880

tattgggtac gatggaacac actttgttga gaactactat ccttatctaa caactattag
5940

gcaacctatt aaagatatcg cacatctttt ggtagacgta ttgctaaaga aaattgatca
6000

ccaagatact cctaaagatt atattctccc cgttggtctt ttatcaggag aaagtgttta
6060

gaatcaaaag gtttatttag gtttgtcttt tgtgggttga ttaaaagtcc ttacctaatt
6120

aatgagcatc agttcaatgt caatatttaa tcaactttgc gcaaaagaat agacatacaa
6180

ttcttagttt taaattcaaa aagaagaggt aagatttaag cctaaaatct tacctcttct
6240

tttatcagcc cttattattt tctttcaagc acaaactgac ttaaaacgcc attaataaac
6300

ttactggatt tctcatctga atactttttt gcaatttcaa taatttcatt aagagcaaca
6360

cgttctggtg tttccttatg atatttaatt tcgtataaac ctaaacgcaa caggctctta
6420

tcaatcaaag tcaatcgaga aagcgtccag ccagactagt agggcaaact attaactggc
6480

gaactactta ctctagcttc ccggcaacaa ttaatagact ggatggaggc ggataaagtt
6540

gcaggaccac ttctgcgctc ggcccttccg gctggctggt ttattgctga taaatctgga
6600

gccggtgagc gtgggtctcg cggtatcatt gcagcactgg ggccagatgg taagccctcc
6660

cgtatcgtag ttatctacac gacggggagt caggcaacta tggatgaacg aaatagacag
6720

atcgctgaga taggtgcctc actgattaag cattggtaac tgtcagacca agtttactca
6780

tatatacttt agattgattt aaaacttcat ttttaattta aaaggatcta ggtgaagatc
6840

ctttttgata atctcatgac caaaatccct taacgtgagt tttcgttcca ctgagcgtca
6900

gaccccttaa taagatgatc ttcttgagat cgttttggtc tgcgcgtaat ctcttgctct
6960

gaaaacgaaa aaaccgcctt gcagggcggt ttttcgaagg ttctctgagc taccaactct
7020

ttgaaccgag gtaactggct tggaggagcg cagtcaccaa aacttgtcct ttcagtttag
7080

ccttaaccgg cgcatgactt caagactaac tcctctaaat caattaccag tggctgctgc
7140

cagtggtgct tttgcatgtc tttccgggtt ggactcaaga cgatagttac cggataaggc
7200

gcagcggtcg gactgaacgg ggggttcgtg catacagtcc agcttggagc gaactgccta
7260

cccggaactg agtgtcaggc gtggaatgag acaaacgcgg ccataacagc ggaatgacac
7320

cggtaaaccg aaaggcagga acaggagagc gcacgaggga gccgccaggg ggaaacgcct
7380

ggtatcttta tagtcctgtc gggtttcgcc accactgatt tgagcgtcag atttcgtgat
7440

gcttgtcagg ggggcggagc ctatggaaaa acggctttgc cgcggccctc tcacttccct
7500

gttaagtatc ttcctggcat cttccaggaa atctccgccc cgttcgtaag ccatttccgc
7560

tcgccgcagt cgaacgaccg agcgtagcga gtcagtgagc gaggaagcgg aatatatcct
7620

gtatcacata ttctgctgac gcaccggtgc agcctttttt ctcctgccac atgaagcact
7680

tcactgacac cctcatcagt gccaacatag taagccagta tacactccgc tagcgctgag
7740

gtctgcctcg tgaagaaggt gttgctgact cataccaggc ctgaatcgcc ccatcatcca
7800

gccagaaagt gagggagcca cggttgatga gagctttgtt gtaggtggac cagttggtga
7860

ttttgaactt ttgctttgcc acggaacggt ctgcgttgtc gggaagatgc gtgatctgat
7920

ccttcaactc agcaaaagtt cgatttattc aacaaagcca cgttgtgtct caaaatctct
7980

gatgttacat tgcacaagat aaaaatatat catcatgaac aataaaactg tctgcttaca
8040

taaacagtaa tacaaggggt gttatgagcc atattcaacg ggaaacgtct tgctcgaggc
8100

cgcgattaaa ttccaacatg gatgctgatt tatatgggta taaatgggct cgcgataatg
8160

tcgggcaatc aggtgcgaca atctatcgat tgtatgggaa gcccgatgcg ccagagttgt
8220

ttctgaaaca tggcaaaggt agcgttgcca atgatgttac agatgagatg gtcagactaa
8280

actggctgac ggaatttatg cctcttccga ccatcaagca ttttatccgt actcctgatg
8340

atgcatggtt actcaccact gcgatccccg ggaaaacagc attccaggta ttagaagaat
8400

atcctgattc aggtgaaaat attgttgatg cgctggcagt gttcctgcgc cggttgcatt
8460

cgattcctgt ttgtaattgt ccttttaaca gcgatcgcgt atttcgtctc gctcaggcgc
8520

aatcacgaat gaataacggt ttggttgatg cgagtgattt tgatgacgag cgtaatggct
8580

ggcctgttga acaagtctgg aaagaaatgc ataagctttt gccattctca ccggattcag
8640

tcgtcactca tggtgatttc tcacttgata accttatttt tgacgagggg aaattaatag
8700

gttgtattga tgttggacga gtcggaatcg cagaccgata ccaggatctt gccatcctat
8760

ggaactgcct cggtgagttt tctccttcat tacagaaacg gctttttcaa aaatatggta
8820

ttgataatcc tgatatgaat aaattgcagt ttcatttgat gctcgatgag tttttctaat
8880

cagaattggt taattggttg taacactggc agagcattac gctgacttga cgggacggcg
8940

gctttgttga ataaatcgaa cttttgctga gttgaaggat cagatcacgc atcttcccga
9000

caacgcagac cgttccgtgg caaagcaaaa gttcaaaatc accaactggt ccacctacaa
9060

caaagctctc atcaaccgtg gctccctcac tttctggctg gatgatgggg cgattcaggc
9120

ctggtatgag tcagcaacac cttcttcacg aggcagacct cagcgctcaa agatgcaggg
9180

gtaaaagcta accgcatctt taccgacaag gcatccggca gttcaacaga tcgggaaggg
9240

ctggatttgc tgaggatgaa ggtggaggaa ggtgatgtca ttctggtgaa gaagctcgac
9300

cgtcttggcc gcgacaccgc cgacatgatc caactgataa aagagtttga tgctcagggt
9360

gtagcggttc ggtttattga cgacgggatc agtaccgacg gtgatatggg gcaaatggtg
9420

gtcaccatcc tgtcggctgt ggcacaggct gaacgccgga ggatcctaga gcgcacgaat
9480

gagggccgac aggaagcaaa gctgaaagga atcaaatttg gccgcaggcg taccgtggac
9540

aggaacgtcg tgctgacgct tcatcagaag ggcactggtg caacggaaat tgctcatcag
9600

ctcagtattg cccgctccac ggtttataaa attcttgaag acgaaagggc ctcgtgatac
9660

gcctattttt ataggttaat gtcatgataa taatggtttc ttagacgtca ggtggcactt
9720

ttcggggaaa tgtgcgcgga acccctattt gtttattttt ctaaatacat tcaaatatgt
9780

atccgctcat gagacaataa ccctgataaa tgcttcaata atattgaaaa aggaagagta
9840

## Description

### BACKGROUND OF THE INVENTION

### 1. Field of the Invention

The present invention relates to a microorganism belonging to the genus Escherichia sp. having a sucrose assimilability and an L-amino acid producing ability, which is obtained by introducing a gene encoding a sucrose assimilative microorganism-derived sucrose metabolic enzyme to a sucrose non-assimilative microorganism belonging to the genus *Escherichia* sp. having an L-amino acid producing ability, and a method for producing an L-amino acid using the same.

### 2. Description of the Related Art

Due to the growing demand for bio-fuel production and crop failures caused by unusual climate, the price of starch sugar used as a main carbon source in industrial fermentation has rapidly increased. Alternatively, the use of sucrose or molasses containing a high concentration of sucrose, cheaper than starch sugar, as a carbon source in industrial fermentation, is advantageous to ensure cost competitiveness.

Approximately 50% of wild-type naturally occurring *E. coli* is able to metabolize sucrose, but E. coli K12 strain, B strain, and C strain usually used in industrial fermentation, have no ability to assimilate sucrose (Mol. Microbiol. (1998) 2:1-8, Can. J. Microbiol. (1999) 45:418-422). Therefore, one of the most important challenges in the fermentation industry is the identification of genes involved in sucrose assimilation, the establishment of enhanced sucrose assimilation-related genes by improvement, and the application of the genes to the sucrose non-assimilative, industrial *E. coli* strains for the production of desired metabolites.

To impart a sucrose-assimilability to industrial *E. coli* strains, methods of introducing genes or gene cluster involved in sucrose assimilation, derived from microorganisms having a sucrose-assimilability have been generally used. For example, a method of imparting sucrose-assimilability to *E. coli* K12 by transformation with the *scr* regulon that is present in the species Salmonella belonging to the family Enterobacceriaceae (J. Bacterial. (1982) 151:68-76, Mol. Microbiol. (1998) 2:1-8, J. Bacteriol. (1991) 173:7464-7470, US Patent No. 7179623), *Klebsiella* pneumoniae (J. Gen. Microbiol. (1988) 134:1635-1644), and *Erwinia amylovora* (J. Bacteriol. (2000) 182:5351-5358) has been well known in the art. Introduction of the csc regulon derived from non-K12 *E. coli* or pathogenic *E. coli* having the sucrose-assimilability (Appl. Environ. Microbiol. (1992) 58:2081-2088, US Patent No. 6960455), introduction of gene cluster involved in sucrose assimilation that is present in conjugative plasmid scr53 isolated from *E. coli* AB1281 (US Patent No. 4806480), and introduction of *scr* regulon and sac operon derived from Gram-positive microorganisms, Streptococcus mutans (J. Bacteriol. (1989) 171:263-271) and Bacillus subtilis (J. Bacteriol. (1989) 171:1519-1523) are also knows. US Patent No. 7179623 discloses a method of producing lysine, isoleucine and valine using *E. coli* K12 that is prepared by introducing an *E. coli* VKPM B-7915-derived *scr* regulon thereto.

However, there is still a need of an industrial microorganism having an efficient sucrose utilization system and a fermentation method using the same. Therefore, the present inventors found that an L-amino acid can be produced from sucrose at a high yield using an L-amino acid-producing microorganism belonging to the genus Escherichia sp. microorganism, which is prepared by introducing a gene cluster involved in sucrose assimilation, derived from a sucrose assimilative *Streprococcus mutans*, thereby completing the present invention.

### SUMMARY OF THE INVENTION

An object of the present invention is to provide a microorganism belonging to the genus *Escherichia* sp. having sucrose assimilability and an L-amino acid producing ability, which is prepared by imparting sucrose assimilability to a sucrose non-assimilative microorganism belonging to the genus *Escherichia* sp. having an L-amino acid producing ability.

Another object of the present invention is to provide a method for producing an L-amino acid from sucrose using the microorganism belonging to the genus *Escherichia* sp. having sucrose assimilability and an L-amino acid producing ability.

### BRIEF DESCRIPTION OF THE DRAWINGS

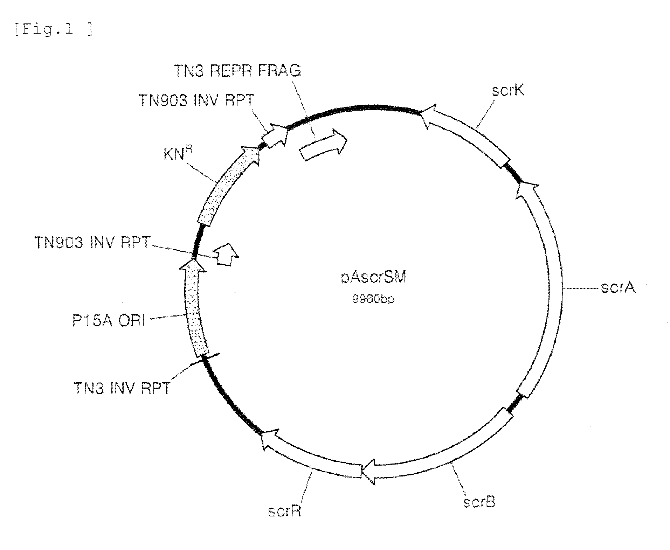

FIG.1 shows the construction of a recombinant plasmid pAscrSM containing Streptococcus *mutans*-derived *scrKABR* according to one specific embodiment of the present invention.

### DETAILED DESCRIPTION OF THE PREFERRED EMBODIMENT

In order to achieve the above objects, the present invention providers a microorganism belonging to the genus *Escherichia* sp. having a sucrose assimilability and an L-amino acid producing ability, which is obtained by introducing a gene encoding a sucrose assimilative microorganism-derived sucrose metabolic enzyme to a sucrose non-assimilative a microorganism belonging to the genus *Escherichia* sp. having an L-amino acid producing ability.

As used herein, the term "sucrose non-assimilative microorganism means a microorganism that cannot utilize sucrose as a carbon source, and the term "microorganism having sucrose assimilability and an L-amino acid producing ability" means a microorganism that can metabolize sucrose as a carbon source so as to produce an L-amino acid.

As used herein, the term "sucrose metabolic enzyme" means an enzyme required for utilization of sucrose as a carbon source, and it includes fructokinase, sucrose PTS permease, sucrose hydrolase, invertase or the like, but is not limited thereto.

In the present invention, the sucrose metabolic enzyme" is also called "Scr-PTS enzyme".

A metabolic system utilizing sucrose as a carbon source can be largely divided into PTS (phosphoenolpyruvate dependent sucrose phosphotransferase)-based sucrose metabolic system (Scr-PTS system) and non-PTS-based sucrose metabolic system (Scr-non PTS system) according to a phosphate source for phosphorylation of influent sucrose in a cell. Most microorganisms capable of utilizing sucrose have the Scr-PTS system.

A representative example of the PTS-based SCAR-PUTS system using phosphoenolpyruvate (PEP) as a phosphate source for phosphorylation of sucrose includes a conjugative plasmid pUP400 of Gram-negative *Salmonella typhimurium*, a *scr* regulon present on the chromosome of *Klebsiella pneumoniae* or the like. The *scr* regulon is composed of 5 genes, *scrK* (fructokinase), *scrY* (sucrose porin), *scrA* (sucrose-specific EIIBC component), *scrB* (sucrose-6-phosphate hydrolase) and *scrR* (LacI-related sucrose-specific repressor), and two operons, *scrK* and *scrYAB* are negatively controlled by the Scar repressor (Mol. Microbiol. (1993) 9:195-209). According to a mechanism of the *scr* regulon, external sucrose is transported into a periplasmic space through an outer membrane protein (OMP), ScrY. The transported sucrose is transported into a cell in the form of sucrose-6-phosphate through a sucrose PTS cycle including ScrA. Sucrose-6-phosphate is then hydrolyzed to glucose-6-phosphate and fructose which are metabolized by ScrB, and fructose is converted into fructose-6-phosphate by an ATP-dependent ScrK, and the resulting fructose-6-phosphate is metabolized via glycolysis, together with glucose-6-phosphate (J. Biotechnol. (2001) 92:133-158). The sucrose PTS cycle, which functions to convert sucrose into sucrose-6-phosphate and then transports it into the cell, is composed of Enzyme I (EI), histidine protein (HPr), glucose-specific enzyme IIA (EIIAcrr^{Glc}), and sucrose-specific enzyme IIBC (EIIBC^{scr}) (J. Biotechnol. (2001) 92:133-158/ J. Biotechnol. (2004) 110:181-199).

Gram-positive microorganism have a variety of Scr-PTS systems, compared to Gram-negative microorganisms. As the Scr-PTS system of *Bacillus subtilis*, a *sac* operon composed of *sacA* (sucrose-6-phosphate hydrolase), *sacP* (sucrose-specific EIIBC component), and *sacT* (transcriptional antiterminator) is well known (J. Bactreriol. (1989) 171:1519-1523). *Corynebacterium glutamicum has* an incomplete system, in which it utilizes sucrose via *ptsS* (sucrose-specific EII component) and *scrB* (sucrose-6-phosphate hydrolase), but free fructose produced from hydrolysis of sucrose-6-phosphate by ScrB is not utilized within the cell, and transported out of the cell, and thereafter the fructose re-enters the cell via the fructose PTS system (J. Mol. Microbiol. Biotechnol. (2007) 12:43-50). Another example of the Scr-PTS system of a Gram-positive microorganism is the *scar* regulon of *Streptococcus mutans*. The *scr* regulon of *Streptococcus mutans* is composed of 4 genes, *scrK* (fructokinase), *scrA* (sucrose-specific EIIABC component), *scrB* (sucrose-6-phosphate hydrolase) and *scrR* (LacI-related sucrose-specific repressor), and *scrA* and *scrB* are negatively controlled by the ScrR repressor (J. Bacteriol. (2003) 185:5791-5799). The *scr* regulon of Streptococcus mutans is characterized by possessing a complete Scr-PTS system having even the fructokinase and sucrose transcriptional regulator, unlike Bacillus subtilis or *Corynebacterium glutamicum*. Therefore, the Scr-PTS system of Streptococcus mutans can be readily introduced into the sucrose non-assimilative strain.

A mechanism of the *scr* regulon of Streptococcus sp. microorganism is as follows. External sucrose is transported into the cell in the form of sucrose-6-phosphate via ScrA. Sucrose-6-phosphate is then hydrolyzed to glucose-6-phosphate and fructose which are metabolized by ScrB, and fructose is converted into fructose-6-phosphate by an ATP-dependent ScrK, and the resulting fructose-6-phosphate is metabolized via glycolysis, together with glucose-6-phosphate (J. Bacteriol. (1986) 166:426-434, FEMS Microbiol. Lett. (1991) 79:339-346). In particular, most Gram-positive microorganisms including Streptococcus sp. microorganism have different characteristics of ScrA from that of Gram-negative *Enterobacteriaceae* sp.. The ScrA of *Enterobacteriaceae* sp. retains activities of Enzyme I (EI), histidine protein (HPr), and glucose specific enzyme IIA (EIIAcrr^{Glc}) as a sucrose PTS cycle (J. Biotechnol. (2004) 110:181-199). However, the ScrA of *Streptococcus mutans* also has a function of enzyme IIA, and thus is not needed to retain the activity of glucose specific enzyme IIA when it is introduced into *Escherichia* sp.. Practically, when the wild-type strains having an inactivated *crr* gene encoding the glucose specific enzyme IIA are produced with each of the *scr* regulons of Streptococcus mutans and *Klebsiella pneumoniae* and they are spread on a MacConkey agar plate containing 1% sucrose, the strain including the *Streptococcus mutans*-derived *scr* regulon shows deep purple colonizes, but the strain including the *Klebsiella pneumoniae*-derived *scr* regulon does not show deep purple colonies.

The Scr-non PTS system, which requires no PTS for uptake of sucrose into the cell, is exemplified by the well know csc regulon. The *csc* regulon is mainly derived from a sucrose-assimilative *E. coli*, and exemplified by csc regulon from the wild type *E.coli* EC3132 (Mol. Gen. Genet. (1992) 235:22-32, US Patent No. 6960455), *csc* regulon from *E.coli* KO11 (Biotechnol. Lett. (2004) 26:689-693), csc regulon from the pathogenic *E.coli* O157:H7 (J. Bacteriol. (2002) 184:5307-5316), *csc* regulon from ATCC13281 (Appl. Microbial. Biotechnol. (2007) 74:1031-1040) or the like. The *csc* regulon consists of *cscB* (proton symport-type sucrose pertease), *cscK* (fructokinase), *cscA* (sucrose hydrolase), and *cscR* (LacI-related sucrose-specific repressor), and two operons, cscKB and *cscA* are negatively controlled by CscR (J. Bacteriol. (2002) 184: 5307-5316).

It was reported that since the Scr-non PTS system is not efficient for uptake of a low level of sucrose, *E.coli* introduced with the csc regulon has a doubling time of 20 hrs in a medium containing sucrose of 0.2% or less (J. Bacteriol. (2002) 184:5307-5316). Unlike the Scr-non PTS system, the Scr-PTS system allows efficient uptake of even a low level of sucrose into the cell. While the uptake of external sucrose by CscB of the Scr-non PTS system is driven by a hydrogen gradient, the Scr-PTS system requires PEP used as an energy source for the uptake of sucrose into the cell, and thus allows efficient uptake of even a low level of sucrose.

In a specific embodiment of the present invention, the gene encoding a sucrose assimilative microorganism-derived sucrose metabolic enzyme is a gene derived from a microorganism having a sucrose-assimilability, and preferably a gene derive from a microorganism having a PTS-based Scr-PTS system.

In a specific embodiment of the present invention, the gene encoding a sucrose assimilative microorganism-derived sucrose metabolic enzyme may be a gene derived from sucrose assimilative Streptococcus mutans.

In a specific embodiment of the present invention, the gene encoding a sucrose assimilative microorganism-derived sucrose metabolic enzyme may be a gene derived from sucrose assimilative Streptococcus mutans ATCC700610.

In a specific embodiment of the present invention, the gene encoding a sucrose assimilative microorganism-derived sucrose metabolic enzyme may be combinations of the genes encoding fructokinase, sucrose PTS permease, sucrose hydrolase, and sucrose transcriptional regulator, which are derived from Streptococcus mutans.

In a specific embodiment of the present invention, the genes encoding the sucrose assimilative microorganism-derived fructokinase, sucrose PTS permease, sucrose hydrolase, and sucrose transcriptional regulator may be *scrK* of SEQ ID No. 4, *scrA* of SEQ ID NO. 5, *scrB* of SEQ ID NO. 6, and *scrR* of SEQ ID NO. 7, respectively.

For the preparation of the microorganism belonging to the genus *Escherichia* sp. having a sucrose assimilability and an L-amino acid producing ability according to the present invention, introduction of the genes encoding the sucrose assimilative microorganism-derived sucrose PTS permease, sucrose hydrolase, fructokinase, and sucrose transcriptional regulator into the sucrose non-assimilative microorganism belonging to the genus *Escherichia* sp. may be performed by the method well known in the art.

In a specific embodiment of the present invention, sequences encoding the sucrose PTS permeate, the sucrose hydrolase, the fructokinase, and the sucrose transcriptional regulator are introduced into a vector to construct a recombinant vector, and the sucrose non-assimilative *Escherichia* sp. microorganism having an L-amino acid producing ability is transformed with the constructed recombinant vector so as to prepare an *Eschberichia* sp. microorganism having a sucrose assimilability and an L-amino acid producing ability.

The vector used for the preparation of the *Escherichia* sp. microorganism of the present invention is not particularly limited, and any known expression vectors may be used. Preferably, pACYC177, pACYC184, pCL, pECCG117, pUC19, pBR322, or pMW118 may be used.

As used herein, the term "transformation" means a method in which a gene is produced into a host cell to be expressed in the host cell. The transformed gene, if it can be expressed in the host cell, may be inserted in the chromosome of the host cell or may exist independent of the chromosome. In addition, the transformed gene is defined as a polynucleotide capable of encoding a polypeptide, and includes DNA and RNA. The transformed gene may be in a suitable form that can be introduced into the host cell and expressed therein. For example, the transformed gene may be introduced into the host cell in the type of expression cassette which is a polynucleotide expressome including whole elements for expressing the gene by itself. Typically, the expression cassette incudes a promoter, a transcription termination signal, a ribosome binding site any a translation termination signal, which are operably linked to the transformed gene. The expression cassette may be in the type of the expression vector capable of self-replication. The transformed gene may also be introduced into the host cell by itself or in the type of polynucleotide expressome so as to be operable linked to the sequence required for expression in the host cell.

In a specific embodiment of the present invention, the sucrose non-assimilative *Escherichia* sp. microorganism having an L-amino acid producing ability may be transformed with a recombinant vector harboring a gene encoding a *Streptococcus mutans*-derived Scr-PTS enzyme in order to acquire a sucrose assimilability.

In a specific embodiment of the present invention, the sucrose non-assimilative *Escherichia* sp. microorganism having an L-amino acid producing ability may be transformed with a recombinant plasmid including a sequence of SEQ ID NO. 8 in order to acquire a sucrose assimilability. Specifically, the recombinant plasmid of SEQ ID NO. 8 includes *Streptococcus mutans* (ATCC700610)-derived *scrKABR,* namely, the fructokinase-encoding *scrK* of SEQ ID NO. 4, the sucrose PTS permease-encoding scrA of SEQ ID NO. 5, the sucrose hydrolase-encoding *scrB* of SEQ ID NO. 6, and the sucrose transcriptional regulator-encoding *scrR* of SEQ ID NO. 7.

The microorganism belonging to the genus *Escherichia* sp. having a sucrose assimilability and an L-amino acid producing ability according to the present invention is a microorganism belonging to the genus *Escinerichia* sp. that is able to produce L-aminio acid and retains the activities of sucrose PTS permease, sucrose hydrolase, fructokinase, and sucrose transcriptional regulator at the same time, so as to utilize sucrose as a carbon source, and preferably *Escheichia coli.*

In a specific embodiment of the present invention, the L-amino acid may bye -threonine, O-succinyl-homoserine, O-acetyl-homoserine, L-methionine, L-lysine, L-homoserine, L-isoleucine, L-valine, or L-tryptophan.

In a specific embodiment of the present invention, the L-amino acid may be L-threonine.

In a specific embodiment of the present Invention, the microorganism belonging two the genus *Escherichia* sp. having a sucrose assimilability and an L-amino acid producing ability may be *Escheichia coli* CA03-0208 (KCCM 10994) that is obtained by transforming *Escheichia coli* ABA5G having an L-threonine-producing ability with a vector having the sequence of SEQ ID NO. 8 including the *Streptococcus mutans-*derived *scrKABR* gene cluster.

Further, the present invention provides a method for producing an L-amino acid, comprising the steps of culturing the microorganism belonging to the genus *Escherichia* sp. having a sucrose assimilability and an L-amino acid producing ability in a medium containing sucrose as a carbon source; and recovering an L-amino acid from the culture medium.

In a specific embodiment of the present invention, the L-amino acid may be L-threonine, O-succinyl-homoserine, O-acetyl-homoserine, L-methionine, L-lysine, L-homoserine, L-isoleucine, L-valine, or L-tryptophan.

In a specific embodiment of the present invention, the L-amino acid may be L-threonine.

The method for producing an L-amino acid according to the present invention include the step of culturing the microorganism belonging to the genus *Escherichia* sp. Having a sucrose assimilability and an L-amino acid producing ability.

In a specific embodiment of the present invention, the step of culturing the *Escherichia* sp. microorganism may be conducted in a medium and under culture conditions that are suitable for the corresponding microorganism. The medium and culture conditions suitably for the corresponding microorganism can be readily selected and adjusted by any person skilled in the art to which it pertains. Examples of the culturing method include batch type, continuous type and fed-batch type manners, but are not limited thereto.

In a specific embodiment of the present invention, the step of culturing the microorganism belonging to the genus *Escherichia* sp. may be conducted buy culturirig the strain in a typical medium that is supplemented with appropriate carbon sources including sucrose, nitrogen sources, amino acids, and vitamins under aerobic conditions and temperature or pH control.

The medium used in the present invention include sucrose or molasses containing a high concentration of sucrose as a main carbon source, and may include various carbon sources in addition to the main carbon source. The nitrogen source included in the medium may be used either singly or in combinations of organic nitrogen sources such as peptone, yeast extract, broth, malt extract, corn steep liquor, and soy bean, and inorganic nitrogen sources such as urea, ammonium sulfate, ammonium chloride, ammonium phosphate, ammonium carbonate and ammonium nitrate. In the medium, phosphorus sources such as potassium dihydrogen phosphate, dipotassium hydrogen phosphate or corresponding sodium-containing salts may be included. In addition, the medium may be supplemented with amino acids, vitamins, and appropriate precursors. These main components may be added to the media in a batch type or a continuos type.

During cultivation, compound such as ammonium hydroxide, potassium hydroxide, ammonia, phosphoric acid, and sulfuric acid may be properly added so as to adjust the pH of the cultures. During cultivation, defoaming agent such as fatty acid polyglycol ester may be used so as to present the formation of foams. Generally, the cultivation temperature may be maintained at 27°C to 37°C, and preferably at 30°C to 35°C. The cultivation may be continued as long as a production amount of the desired material, L-amino acid is increased under the given conditions, and for example, for 10 to 100 hrs.

The method for producing an L-amino acid according to the present invention includes the step of recovering the L-amino acid from the culture of the microorganism. The method of recovering the L-amino acid from the culture may be performed by a proper method known in the art, depending on the culturing procedures, for example, batch type, continuos type or fed-batch type.

Hereinafter, the present invention will be described in more detail with reference to Examples. However, these Examples are for illustrative purposes only, and the invention is not intended to be limited by these Examples.

### Example 1: Cloning and Identification of Sequence of scr regulon which is involved in sucrose assimilation

### (1) Cloning of sucrose assimilative microorganism-derived scr regulon

To impart a sucrose assimilability to a sucrose non-assimilative *Escherichia* sp. microorganism, the gene cluster involved in sucrose assimilation, *scr* regulon was obtained from a sucrose assimilative microorganism, *Streptococcus mutans.* The *scr* regulon of Gram-positive *Streptococcus mutans* is composed of four genes, *scrK* (fructokinase), *scrA* (sucrose-specific EIIBC component), *scrB* (sucrose-6-phosphate hydrolase), and *scrR* (LacI-related sucrose-specific repressor), and two operons, *scrA* and *scrB* are negatively controlled by the ScrR repressor (J. Bacteriol. (2003) 185:5791-5799). In particular, the *scr* regulon of *Streptococcus mutans* is characterized by possessing a complete Scr-PTS system having the fructokinase and sucrose transcriptional regulator, unlike *Bacillus subtilis* or *Corynebacterium glutamicum.*

The Scr-PTS system, *scrKABR* genes were obtained by PCR (Polymerase Chain Reaction) using the chromosome of *Streptococcus mutans* (ATCC700610D-5) purchased from American Type Culture Collection as a template.

The *scrKABR* gene that is the *scr* regulon of *Streptococcus mutans* was amplified by PCR using a pair of primers of SEQ ID NO. 1 and SEQ ID NO. 2, so as to obtain four types of genes, which are consecutively present on the genome, as a single polynucleotide. The primer of SEQ ID NO. 1 has the *ApaLI* restriction site, and the primer of SEQ ID NO.2 has the *StuI* restriction site. The primers that were used were prepared based on information on *scrKABR* and its surrounding sequence of *Streptococcus mutans* (KEGG organism, smu) available in the KEGG (Kyoto Encyclopedia of Genes and Genomes).

PCR was performed under the conditions including denaturation at 94°C for 3 min, 25 cycles of denaturation at 94°C for 30 sec, annealing at 56°C for 30 sec, and polymerization of at 72°C for 5 min, and then polymerization for at 72°C for 7 min. As a result, a polynucleotide of 6480 bp was obtained from *Streptococcus mutans.* The polynucleotide obtained by PCR was treated with *ApaLI* and *FspI,* and then cloned into the *ApaLI* any *FspI* sites of a pACYC177 vector. Thereafter, E. *coli* DH5α was transformed with the vector, and spread on a NacConkey agar plate containing 1% sucrose. Among the colonies, deep purple colonies were selected, and then a plasmid derived from *Streptococcus* mutans was obtained using a typical plasmid miniprep.

### (2) Identification of sequence determination of scrKABR gene

The plasmid containing the *scr* regulon of *Streptococcus mutans* obtained in (1) was designated as pAscrSM, and the sequence (SEQ ID NO. 3) of *scrKABR* cloned into the *ApaLI* and *FspI* sites was determined by a sequence determination method typically used in the art. FIG. 1 shows the construction of a recombinant plasmid pAscrSM containing *Streptococcus mutans*-derived *scrKABR.* In the *scrKABR* sequence of SEQ ID NO. 3, the position from 392 to 1273 was identified as the *sccK* gene (SEQ ID NO. 4), the position from 1473 to 3467 was identified as the *scrA* gene (SEQ ID NO.5), the position from 3663 to 5102 was identified as the scrB gene (SEQ ID NO. 6), and the position from 5105 to 6067 was identified as the *scrR* gene (SEQ ID NO. 7).

### Examples 2: Construction of sucrose assimilative, L-amino acid producing microorganism

### (1) Transformation with recombinant plasmid

In order to examine whether a threonine-producing *E*. *coli* grows using sucrose and produces threonine efficiently, when the *E. coli* is transformed with the gene cluster involved in sucrose assimilation, *scr* regulon-containing pAscrSM (SEQ ID NO. 8) obtained in Example 1, the plasmid was introduced into *E. coli* ABA5G by a typical transformation method. The *E. coli* ABA5G transformed with pAscrSM was spread on a MacConkey agar plate containing 1% sucrose. Among the colonies, deep purple colonies were selected. PCR was performed to conform that the selected colonies had the sucrose assimilation related gene-containing plasmid.

### (2) Production of threonine by microorganism transformed with pAscrSM

The colony obtained in (1) was cultured on a LB solid medium (1 g of tryptone, 1 g of NaCl, 0.5 g/100 ml of yeast extract, 1.5% agar) in a 33°C incubator overnight. One loop of the cultured strain was inoculate in 2.5 mL of a titration medium having the composition of the following Table 1 and sucrose as a main carbon source, and then cultured in a 33°C incubator at 200 rpm for 90 hrs.

**[Table 1]**

| Composition | Concentration (per liter) |
|---|---|
| Sucrose | 70 g |
| KH₂PO₄ | 2 g |
| (NH₄)₂SO₄ | 25 g |
| MgSO₄-7H₂O | 1 g |
| FeSO₄·7H₂O | 5 mg |
| MnSO₄·4H₂O | 5 mg |
| Yeast extract | 2 g |
| Calcium carbonate | 30 g |
| pH | 6.8 |

As a control group, the parental strain *E. coli* ABA5G transformed with no plasmid was used, and cultured in the medium having the composition of Table 1 using glucose instead of sucrose, in order to compare the sucrose utilization rate to the glucose utilization rate and threonine productivity. The results are summarized in the following Table 2.

**[Table 2]**

| | Glucose (70 g/L) | | Sucrose (70 g/L) | |
|---|---|---|---|---|
| | OD | L-threonine (g/L) | OD | L-threonine (g/L) |
| ABA5G | 14.2 | 21.2 | - | - |
| ABA5G/pAscrSM | 12.1 | 17.4 | 13.2 | 21.7 |

As shown in Table 2, the pAscrSM-harboring *E. coli* ABASG/pAscrSM produced 17.4 g/L of L-threonine in the titration medium containing glucose, and produced 21.7 g/L of L-threonine in the nitration medium containing sucrose during 90 hr cultivation. On the contrary, the parental strain *E. coli* ABA5G transformed with no plasmid produced 21.2 g/L of L-threonine by utilizing glucose during 90 hr cultivation, but did not grow in the medium containing sucrose as a sole carbon source. This result indicates that the sucrose non-assimilative *E. coli* ABA5G having an L-threonine producing ability is produced with pAscrSM so as to have sucrose assimilability, and therefore it utilizes sucrose to have L-threonine productivity equivalent to or better than that of the parental strain E.coli ABA5G utilizing the glucose titration medium.

Therefore, the pAscrSM-harboring recombinant strain showed excellent sucrose utilization and L-threonine productivity, and thus the transformed microorganism was designated as CA03-0208, deposited in the international depository authority, Korean Culture Center of Microorganisms, which is the Subsidiary Culture Collection of the Korean Federation of Culture Collections, (located at 361-221, Hongje-1-dong, Seodaemon-gu, Seoul, Korea) on Feb. 23, 2009, and assigned accession number KCCM 10994.

It will be apparent to those skilled in the art that various modifications and changes may be made thereto without departing from the scope and spirit of the invention. Therefore, it should be understood that the above embodiments are not limitative, but illustrative in all aspects.

The sequences of SEQ ID NOs. 1 to 8 described herein are listed in the accompanying sequence listing.

### Effect of the invention

The microorganism having a sucrose assimilability and an L-amino acid producing ability according to the present invention is used to economically produce an L-amino acid using inexpensive sucrose as a carbon source.

## Claims

1. A microorganism belonging to the genus *Escherichia* sp. having a sucrose assimilability and an L-amino acid producing ability, which is obtained by introducing *Streptococcus mutans*-derived genes encoding fructokinase, sucrose PTS permease, sucrose hydrolase, and sucrose transcriptional regulator into a sucrose non-assimilative microorganism belonging to the genus *Escherichia* sp. having an L-amino acid producing ability.

2. The microorganism belonging to the genus *Escherichia* sp. according to claim 1, wherein the genes encoding fructokinase, sucrose PTS permease, sucrose hydrolase, and sucrose transcriptional regulator are *scrK* of SEQ ID NO. 4, *scrA* of SEQ ID NO. 5, *scrB* of SEQ ID NO. 6, and *scrR* of SEQ ID NO. 7, respectively.

3. The microorganism belonging to the genus *Escherichia* sp. according to claim 2, wherein the microorganism belonging to the genus *Escherichia* sp. is obtained by transforming the sucrose non-assimilative microorganism belonging to the genus *Escherichia* sp. with a recombinant vector of SEQ ID NO. 8.

4. The microorganism belonging to the genus *Escherichia* sp. according to claim 1, wherein the microorganism belonging to the genus *Escherichia* sp. is *E. coli*.

5. The microorganism belonging to the genus *Escherichia* sp. according to claim 4, wherein the *E.* coli is *E. coli* CA03-0208 (KCCM 10994).

6. The microorganism belonging to the genus *Escherichia* sp. according to claim 1, wherein the L-amino acid is L-threonine.

7. A method for producing an L-amino acid, comprising the steps of culturing the microorganism belonging to the genus *Escherichia* sp. as in one of claims 1-6 in a medium containing sucrose as a carbon source; and recovering an L-amino acid from the culture medium.

8. The method according to claim 7, wherein the L-amino acid is L-threonine.
